(19) Europäisches Patentamt
European Patent Office
Office européen des brevets

(11) EP 4 635 473 A1

(12) EUROPEAN PATENT APPLICATION
published in accordance with Art. 153(4) EPC

(43) Date of publication:
**22.10.2025 Bulletin 2025/43**

(21) Application number: **23903560.3**

(22) Date of filing: **14.12.2023**

(51) International Patent Classification (IPC):
***A61K 8/68*** *(2006.01)* ***A61K 8/06*** *(2006.01)*
***A61K 8/34*** *(2006.01)* ***A61K 8/44*** *(2006.01)*
***A61K 8/46*** *(2006.01)* ***A61K 8/86*** *(2006.01)*
***A61Q 19/00*** *(2006.01)*

(52) Cooperative Patent Classification (CPC):
**A61K 8/02; A61K 8/06; A61K 8/34; A61K 8/42; A61K 8/44; A61K 8/46; A61K 8/68; A61K 8/86; A61Q 19/00**

(86) International application number:
**PCT/JP2023/044789**

(87) International publication number:
**WO 2024/128276 (20.06.2024 Gazette 2024/25)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **14.12.2022 JP 2022199684**

(71) Applicant: **Kao Corporation**
**Chuo-ku,**
**Tokyo 103-8210 (JP)**

(72) Inventor: **AKAHANE, Tomoki**
**Odawara-shi, Kanagawa 250-0002 (JP)**

(74) Representative: **Hoffmann Eitle**
**Patent- und Rechtsanwälte PartmbB**
**Arabellastraße 30**
**81925 München (DE)**

(54) **OIL-IN-WATER EMULSION COMPOSITION**

(57) A transparent or translucent oil-in-water emulsified composition comprising following components (A), (B), (C), (D), (E), and (F): (A) a ceramide; (B) an anionic surfactant selected from the group consisting of N-acyl-N-alkyltaurine, N-acyl-L-glutamic acid, and salts thereof; (C) a nonionic surfactant having an HLB of 10 or more; (D) a dihydric alcohol having 6 to 10 carbon atoms; (E) a dihydric alcohol having 5 or less carbon atoms; and (F) water.

EP 4 635 473 A1

## Description

Field of the Invention

The present invention relates to an oil-in-water emulsified composition.

Background of the Invention

Lipids referred to as stratum corneum lipids are present in gaps between corneocytes which compose stratum corneum, which is present in the outermost layer of skin. Ceramide, which accounts for approximately 50% of the stratum corneum lipids, is deeply related to rough skin and dry skin, and it is known that the condition of the stratum corneum may be improved by externally supplementing the ceramide.

Where solid lipids such as a ceramide are blended into cosmetics, since solid lipids are highly crystalline and have high melting points, it is difficult to stabilize solid lipids in formulations. Therefore, techniques for stably blending solid lipids have been studied.

For example, Patent Literature 1 describes that, in a dispersion of fine lipid particles comprising one or more selected from the group consisting of a sterol and a derivative thereof; a lipid which is solid or semi-solid at 25°C such as a ceramide; and an anionic surfactant, the solid lipid such as a ceramide may be stably emulsified and dispersed as fine particles without being crystallized.

Patent Literature 2 describes that a composition in the form of a nano- or micro-emulsion comprising a nonionic surfactant having an HLB of from 8 to 14, a ceramide compound, and vitamin B3 or derivatives thereof may enhance or improve the penetration of the ceramide compound into skin.

(Patent Literature 1) JP-A-2019-119742
(Patent Literature 2) JP-A-2018-87149

Summary of the Invention

The present invention relates to a transparent or translucent oil-in-water emulsified composition comprising following components (A), (B), (C), (D), (E), and (F):

(A) a ceramide;
(B) an anionic surfactant selected from the group consisting of N-acyl-N-alkyltaurine, N-acyl-L-glutamic acid, and salts thereof;
(C) a nonionic surfactant having an HLB of 10 or more;
(D) a dihydric alcohol having 6 to 10 carbon atoms;
(E) a dihydric alcohol having 5 or less carbon atoms; and
(F) water.

Detailed Description of the Invention

Although the compositions of Patent Literatures 1 and 2 are transparent at room temperature immediately after production, when the compositions are frozen and then melted again, the compositions become cloudy due to aggregation of emulsified particles. It has been necessary to blend a polyol or a thickener in order to prevent this, but there has been a problem that the blending limits use impression.

The inventor found the fact that an oil-in-water emulsified composition comprising a ceramide stably emulsified and dispersed as fine particles in a general-purpose process and having a transparent or translucent appearance which is excellent in storage stability at low and high temperatures and which imparts a fresh feeling and smoothness to skin after application while preventing deterioration in appearance due to freezing and melting may be obtained by using a dihydric alcohol having 6 to 10 carbon atoms and a dihydric alcohol having 5 or less carbon atoms in combination together with the ceramide, a specific anionic surfactant, and a specific nonionic surfactant.

The oil-in-water emulsified composition of the present invention comprises a ceramide stably emulsified and dispersed as fine particles in a general-purpose process and has a transparent or translucent appearance.

Examples of the ceramide as the component (A) used for the present invention include natural ceramides such as ceramides 1, 2, 3, 4, 5, 6, and 7; and sphingosine derivatives such as phytosphingosine, as well as ceramide-like structure substances described in JP-A-62-228048, JP-A-63-216812, JP-A-63-227513, JP-A-64-29347, JP-A-64-31752, JP-A-8-319263, and the like. Specific examples of the ceramide-like structure substance include compounds of the following

formulas (1) and (2) from the viewpoint of enhancing the smoothness of the skin after application and the moisturizing effect.

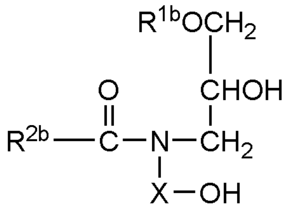

(1)

Wherein $R^{1b}$ represents a hydrocarbon group having 10 to 26 carbon atoms; $R^{2b}$ represents a hydrocarbon group having from 9 to 25 carbon atoms; and X represents - $(CH_2)_n$-, wherein n represents an integer of from 2 to 6.

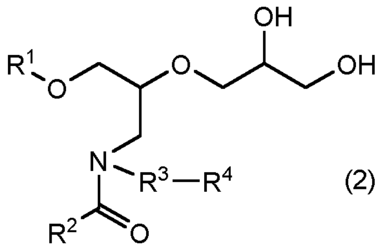

(2)

Wherein $R^1$ and $R^2$ are the same or different and each represent a hydrocarbon group having 1 to 40 carbon atoms which is optionally hydroxylated; $R^3$ represents an alkylene group having 1 to 6 carbon atoms or a single bond; $R^4$ represents a hydrogen atom, an alkoxy group having 1 to 12 carbon atoms, or a 2,3-dihydroxypropyloxy group, provided that $R^4$ is a hydrogen atom when $R^3$ is a single bond.

In the formulas (1) and (2), the hydrocarbon group is preferably an alkyl group or an alkenyl group.

Examples of the compound of the formula (1) include N-(hexadecyloxyhydroxypropyl)-N-hydroxyethylhexadecanamide, and examples of the compound of the formula (2) include long-chain dibasic acid bis 3-methoxypropylamide.

The compound of the formula (1) is a pseudo-ceramide and is a ceramide functional component. The compound may supplement the function of the ceramide and may improve the condition of the skin (such as a moisture content).

From the viewpoint of enhancing the smoothness of the skin after application and the moisturizing effect, the component (A) comprises preferably one or more selected from the group consisting of a natural ceramide, a sphingosine, and the compounds of the formulas (1) and (2); more preferably one or more selected from the group consisting of the natural ceramide, the sphingosine, and the compound of the formula (1); further more preferably one or more selected from the group consisting of the natural ceramide, phytosphingosine, and N-(hexadecyloxyhydroxypropyl)-N-hydroxyethylhexadecanamide; and even more preferably the N-(hexadecyloxyhydroxypropyl)-N-hydroxyethylhexadecanamide.

One or more components may be used for the component (A). From the viewpoint of enhancing the smoothness of the skin after application, a content of the component (A) is preferably 0.000 01 mass% or more, more preferably 0.001 mass% or more, further more preferably 0.005 mass% or more, even more preferably 0.03 mass% or more, particularly preferably 0.125 mass% or more, preferably 0.5 mass% or less, more preferably 0.4 mass% or less, further more preferably 0.35 mass% or less, even more preferably 0.27 mass% or less, and particularly preferably 0.23 mass% or less, with respect to the total composition.

The content of the component (A) is preferably from 0.000 01 to 0.5 mass%, more preferably from 0.001 to 0.4 mass%, further more preferably from 0.005 to 0.35 mass%, even more preferably from 0.03 to 0.27 mass%, and particularly preferably from 0.125 to 0.23 mass%, with respect to the total composition.

The anionic surfactant as the component (B) is selected from the group consisting of N-acyl-N-alkyltaurine, N-acyl-L-glutamic acid, and salts thereof. The N-acyl-N-alkyltaurine and the salt thereof are preferably N-acyl-N-methyltaurine and a salt thereof, and examples thereof include N-myristoyl-N-methyltaurine, N-lauroyl-N-methyltaurine, N-stearoyl-N-methyltaurine, and salts thereof. The salt of N-acyl-N-alkyltaurine is preferably a sodium salt, and examples thereof include sodium N-myristoyl-N-methyltaurine, sodium N-lauroyl-N-methyltaurine, and sodium N-stearoyl-N-methyltaurine. Of these, from the viewpoint of forming the ceramide into fine particles and enhancing the transparency of the appearance, the storage stability at low temperature, and the storage stability at high temperature, the component (B) comprises preferably one or more selected from the group consisting of the N-lauroyl-N-methyltaurine, the N-stearoyl-N-methyltaurine, and the salt thereof; more preferably the N-stearoyl-N-methyltaurine and the salt thereof; and further more preferably the sodium N-stearoyl-N-methyltaurine.

Examples of the N-acyl-L-glutamic acid and the salt thereof include N-lauroyl-L-glutamic acid, N-stearoyl-L-glutamic acid, N-myristoyl-L-glutamic acid, and salts thereof. Of these, from the viewpoint of forming the ceramide into fine particles and enhancing the transparency of the appearance, the storage stability at low temperature, and the storage stability at high temperature, the component (B) comprises preferably one or more selected from the group consisting of the N-lauroyl-L-glutamic acid, the N-stearoyl-L-glutamic acid, and the salt thereof; and more preferably the N-stearoyl-L-glutamic acid and the salts thereof.

From the viewpoint of forming the ceramide into fine particles and enhancing the transparency of the appearance, the storage stability at low temperature, and the storage stability at high temperature, the component (B) comprises preferably the N-acyl-N-methyltaurine and the salt thereof; more preferably one or more selected from the group consisting of the N-lauroyl-N-methyltaurine, the N-stearoyl-N-methyltaurine, and the salt thereof; further more preferably the N-stearoyl-N-methyltaurine and the salt thereof; and even more preferably the sodium N-stearoyl-N-methyltaurine.

One or more components may be used for the component (B). From the viewpoint of forming the ceramide into fine particles and enhancing the transparency of the appearance, the storage stability at low temperature, the storage stability at high temperature, and the smoothness of the skin after application, a content of the component (B) is preferably 0.001 mass% or more, more preferably 0.015 mass% or more, further more preferably 0.023 mass% or more, even more preferably 0.028 mass% or more, particularly preferably 0.048 mass% or more, preferably 0.5 mass% or less, more preferably 0.35 mass% or less, further more preferably 0.2 mass% or less, even more preferably 0.09 mass% or less, and particularly preferably 0.07 mass% or less, with respect to the total composition. The content of the component (B) is preferably from 0.001 to 0.5 mass%, more preferably from 0.015 to 0.35 mass%, further more preferably from 0.023 to 0.2 mass%, even more preferably from 0.028 to 0.09 mass%, and particularly preferably from 0.048 to 0.07 mass%, with respect to the total composition.

In the present invention, from the viewpoint of forming the ceramide into fine particles, enhancing the transparency of the appearance, enhancing the storage stability at low temperature, enhancing the storage stability at high temperature, and suppressing deterioration in the appearance due to freezing and melting, a mass ratio of the component (A) to the component (B), (A)/(B), is preferably 2 or more, more preferably 2.2 or more, further more preferably 2.7 or more, even more preferably 2.9 or more, preferably 7 or less, more preferably 6 or less, further more preferably 5 or less, and even more preferably 4.5 or less. The mass ratio of the component (A) to the component (B), (A)/(B), is preferably from 2 to 7, more preferably from 2.2 to 6, further more preferably from 2.7 to 5, and even more preferably from 2.9 to 4.5.

The nonionic surfactant as the component (C) has an HLB of 10 or more. From the viewpoint of forming the ceramide into fine particles, enhancing the transparency of the appearance and the storage stability at high temperature, and suppressing deterioration in the appearance due to freezing and melting, the HLB is preferably from 11 to 16, and more preferably from 12 to 15.

The HLB (Hydrophilic-Lipophilic Balance) indicates the molecular weight of the hydrophilic group part in the total molecular weight of the surfactant, and is determined by the Griffin equation. Where the component (C) is composed of two or more nonionic surfactants, the HLB of the mixed surfactants is determined in the following manner. The HLB of the mixed surfactants is the arithmetic mean of the HLB values of the nonionic surfactants based on the blending ratio:

$$\text{Mixed HLB} = \Sigma(\text{HLBx} \times \text{Wx})/\Sigma\text{Wx}$$

wherein HLBx represents the HLB value of the nonionic surfactant X, and
wherein Wx represents the mass (g) of the nonionic surfactant X having the HLBx value.

Examples of the nonionic surfactant include polyoxyethylene hydrogenated castor oil, polyoxyethylene sorbitan fatty acid ester, polyoxyethylene alkyl ether, polyoxyethylene fatty acid ester, polyoxyethylene alkanol ether, polyglycerin fatty acid ester, and sucrose fatty acid ester. Of these, from the viewpoint of forming the ceramide into fine particles, enhancing the transparency of the appearance and the storage stability at high temperature, and suppressing deterioration in the appearance due to freezing and melting, the component (C) comprises preferably one or more selected from the group consisting of the polyoxyethylene hydrogenated castor oil and the polyoxyethylene sorbitan fatty acid ester, and more preferably the polyoxyethylene hydrogenated castor oil.

With regard to the polyoxyethylene hydrogenated castor oil, from the viewpoint of forming the ceramide into fine particles, enhancing the transparency of the appearance and the storage stability at high temperature, and suppressing deterioration in the appearance due to freezing and melting, the average number of moles added of polyoxyethylene chains which compose the polyoxyethylene hydrogenated castor oil is preferably from 25 to 100, more preferably from 35 to 85, further more preferably from 45 to 75, even more preferably from 55 to 65, and particularly preferably 60.

With regard to the polyoxyethylene sorbitan fatty acid ester, from the viewpoint of forming the ceramide into fine particles, enhancing the transparency of the appearance and the storage stability at high temperature, and suppressing deterioration in the appearance due to freezing and melting, the average number of moles added of polyoxyethylene chains which compose the polyoxyethylene sorbitan fatty acid ester is preferably from 10 to 30, more preferably from 15 to

25, and further more preferably 20. With regard to the polyoxyethylene sorbitan fatty acid ester, from the viewpoint of forming the ceramide into fine particles, enhancing the transparency of the appearance and the storage stability at high temperature, and suppressing deterioration in the appearance due to freezing and melting, the number of carbon atoms of the alkyl group which compose the polyoxyethylene sorbitan fatty acid ester is preferably from 10 to 25, more preferably from 16 to 20, and further more preferably 18.

One or more components may be used in combination for the nonionic surfactant as the component (C). From the viewpoint of forming the ceramide into fine particles, enhancing the transparency of the appearance, enhancing the storage stability at high temperature, suppressing deterioration in the appearance due to freezing and melting, and enhancing the fresh feeling of the skin after application, a content of the component(C) is preferably 0.1 mass% or more, more preferably 0.13 mass% or more, further more preferably 0.2 mass% or more, even more preferably 0.26 mass% or more, preferably 4 mass% or less, more preferably 2 mass% or less, further more preferably 0.7 mass% or less, and even more preferably 0.35 mass% or less, with respect to the total composition. The content of the component (C) is preferably from 0.1 to 4 mass%, more preferably from 0.13 to 2 mass%, further more preferably from 0.2 to 0.7 mass%, and even more preferably from 0.26 to 0.35 mass%, with respect to the total composition.

The component (D) is a dihydric alcohol having 6 to 10 carbon atoms.

Examples of the component (D) include triethylene glycol, dipropylene glycol, and tripropylene glycol, and the component (D) preferably comprises the dipropylene glycol.

One or more components may be used in combination for the component (D). From the viewpoint of dissolving the ceramide in the production process, forming the ceramide into fine particles, enhancing the transparency of the appearance, enhancing the storage stability at low temperature, and enhancing the fresh feeling of the skin after application, a content of the component(D) is preferably 0.1 mass% or more, more preferably 0.13 mass% or more, further more preferably 0.15 mass% or more, even more preferably 0.3 mass% or more, preferably 10 mass% or less, more preferably 6 mass% or less, further more preferably 3 mass% or less, and even more preferably 1 mass% or less, with respect to the total composition. The content of the component (D) is preferably from 0.1 to 10 mass%, more preferably from 0.13 to 6 mass%, further more preferably from 0.15 to 3 mass%, and even more preferably from 0.3 to 1 mass%, with respect to the total composition.

In the present invention, from the viewpoint of forming the ceramide into fine particles, enhancing the transparency of the appearance, enhancing the storage stability at low temperature, enhancing the storage stability at high temperature, suppressing deterioration in the appearance due to freezing and melting, enhancing the fresh feeling, and enhancing the smoothness of the skin after application, a mass ratio of the component (B) to the component (D), (B)/(D), is preferably 0.005 or more, more preferably 0.01 or more, further more preferably 0.02 or more, even more preferably 0.06 or more, preferably 7 or less, more preferably 1 or less, further more preferably 0.5 or less, and even more preferably 0.2 or less. The mass ratio of the component (B) to the component (D), (B)/(D), is preferably from 0.005 to 7, more preferably from 0.01 to 1, further more preferably from 0.02 to 0.5, and even more preferably from 0.06 to 0.2.

The component (E) is a dihydric alcohol having 5 or less carbon atoms.

Examples of the component (E) include ethylene glycol, diethylene glycol, propylene glycol, and 1,3-butylene glycol. From the viewpoint of forming the ceramide into fine particles, enhancing the transparency of the appearance, enhancing the storage stability at high temperature, suppressing deterioration in the appearance due to freezing and melting, and enhancing the fresh feeling of the skin after application, the component (E) comprises preferably one or more selected from the group consisting of the propylene glycol and the 1,3-butylene glycol, and more preferably the 1,3-butylene glycol.

One or more components may be used in combination for the component (E). From the viewpoint of forming the ceramide into fine particles, enhancing the transparency of the appearance, enhancing the storage stability at low temperature, enhancing the storage stability at high temperature, suppressing deterioration in the appearance due to freezing and melting, and enhancing the fresh feeling of the skin after application, a content of the component(E) is preferably 0.1 mass% or more, more preferably 0.5 mass% or more, further more preferably 1.7 mass% or more, even more preferably 2 mass% or more, preferably 10 mass% or less, more preferably 7.5 mass% or less, further more preferably 2.7 mass% or less, and even more preferably 4 mass% or less, with respect to the total composition. The content of the component (E) is preferably from 0.1 to 10 mass%, more preferably from 0.5 to 7.5 mass%, further more preferably from 1.7 to 2.7 mass%, and even more preferably from 2 to 4 mass%, with respect to the total composition.

In the present invention, from the viewpoint of forming the ceramide into fine particles, enhancing the transparency of the appearance, enhancing the storage stability at high temperature, suppressing deterioration in the appearance due to freezing and melting, and enhancing the fresh feeling of the skin after application, a total content of the components (D) and (E), (D) + (E), is preferably 0.1 mass% or more, more preferably 1 mass% or more, further more preferably 2.3 mass% or more, even more preferably 2.8 mass% or more, particularly preferably 3 mass% or more, preferably 15 mass% or less, more preferably 12 mass% or less, further more preferably 9 mass% or less, even more preferably 5.5 mass% or less, and particularly preferably 5 mass% or less, with respect to the total composition. The total content of the components (D) and (E), (D) + (E), is preferably from 0.1 to 15 mass%, more preferably from 1 to 12 mass%, further more preferably from

2.3 to 9 mass%, even more preferably from 2.8 to 5.5 mass%, and particularly preferably from 3 to 5 mass%, with respect to the total composition.

In the present invention, from the viewpoint of forming the ceramide into fine particles, enhancing the transparency of the appearance, enhancing the storage stability at low temperature, enhancing the storage stability at high temperature, suppressing deterioration in the appearance due to freezing and melting, enhancing the fresh feeling, and enhancing the smoothness of the skin after application, a mass ratio of the component (D) to the component (E), (D)/(E), is preferably 0.01 or more, more preferably 0.02 or more, further more preferably 0.04 or more, even more preferably 0.06 or more, particularly preferably 0.08 or more, more preferably 0.2 or more, preferably 10 or less, more preferably 5 or less, further more preferably 2 or less, even more preferably 1.4 or less, particularly preferably 1 or less, and more preferably 0.56 or less. The mass ratio of the component (D) to the component (E), (D)/(E), is preferably from 0.01 to 10, more preferably from 0.02 to 5, further more preferably from 0.04 to 2, even more preferably from 0.06 to 1.4, particularly preferably from 0.08 to 1, and more preferably from 0.2 to 0.56.

In the present invention, from the viewpoint of enhancing the fresh feeling of the skin after application and the stability at high temperature, a content of the water as the component (F) is preferably 50 mass% or more, more preferably 60 mass%, further more preferably 70 mass% or more, preferably 99 mass% or less, more preferably 95 mass% or less, and further more preferably 90 mass% or less, with respect to the total composition. The content of the water as the component (F) is preferably from 50 to 99 mass%, more preferably from 60 to 95 mass%, and further more preferably from 70 to 90 mass%, with respect to the total composition.

The oil-in-water emulsified composition of the present invention may further comprise (G) a basic amino acid, thereby forming the ceramide into fine particles, enhancing the transparency of the appearance and the storage stability at high temperature, and suppressing deterioration in the appearance due to freezing and melting.

Examples of the basic amino acid include arginine, lysine, and histidine. From the viewpoint of forming the ceramide into fine particles, enhancing the transparency of the appearance and the storage stability at high temperature, and suppressing deterioration in the appearance due to freezing and melting, the oil-in-water emulsified composition preferably comprises the arginine.

One or more components may be used in combination for the component (G). From the viewpoint of forming the ceramide into fine particles, enhancing the transparency of the appearance and the storage stability at high temperature, and suppressing deterioration in the appearance due to freezing and melting, a content of the component (G) is preferably 0.01 mass% or more, more preferably 0.02 mass% or more, further more preferably 0.05 mass% or more, even more preferably 0.1 mass% or more, preferably 1 mass% or less, more preferably 0.8 mass% or less, further more preferably 0.5 mass% or less, and even more preferably 0.2 mass% or less, with respect to the total composition. The content of the component (G) is preferably from 0.01 to 1 mass%, more preferably from 0.02 to 0.8 mass%, further more preferably from 0.05 to 0.5 mass%, and even more preferably from 0.1 to 0.2 mass%, with respect to the total composition.

The oil-in-water emulsified composition of the present invention may comprise, in addition to the above components, components which are usually used in cosmetics such as oil components other than the above components, surfactants other than the above components, water-soluble polymers, antioxidants, fragrances, preservatives, pH-adjusting agents, blood-circulation promoters, cool-feeling agents, antiperspirants, bactericides, skin activators, moisturizers, refreshing agents, and colorants.

With regard to the oil-in-water emulsified composition of the present invention, from the viewpoint of the use impression, a content of oil components other than the component (A) is preferably 15 mass% or less, more preferably 5 mass% or less, further more preferably 1 mass%, even more preferably 0.5 mass% or less, and particularly preferably 0.1 mass% or less.

The oil-in-water emulsified composition of the present invention may be produced in accordance with usual methods.

For example, the components (A) to (E) and other oily components are heated and stirred to prepare an oily-component dissolved phase in which the components are homogeneously dissolved, and then an aqueous phase in which an aqueous component comprising the component (F) has been heated and mixed is added to the oily-component dissolved phase, which is then stirred. Thereafter, the resulting mixture may be cooled to obtain the oil-in-water emulsified composition.

The oil-in-water emulsified composition of the present invention has a transparent or translucent appearance.

Being transparent means that the transmittance is 70% or more with respect to pure water.

Being translucent means that the transmittance is less than 70% and 40% or more with respect to pure water.

In the present invention, the transmittance is calculated as the rate at which light having a wavelength of 550 nm passes through the oil-in-water emulsified composition at 25°C assuming that the transmittance of pure water is 100%.

The oil-in-water emulsified composition of the present invention is excellent in storage stability at low and high temperatures, and may impart a fresh feeling and smoothness to skin after application, while preventing deterioration in appearance due to freezing and melting.

With regard to the oil-in-water emulsified composition of the present invention, from the viewpoint of having a

transparent or translucent appearance and enhancing the storage stability at high temperature, an average particle size of the emulsified particle is preferably 10 nm or more, more preferably 40 nm or more, further more preferably 60 nm or more, even more preferably 90 nm or more, preferably 200 nm or less, more preferably 170 nm or less, further more preferably 135 nm or less, and even more preferably 110 nm or less. The average particle size of the emulsified particle is preferably from 10 to 200 nm, more preferably from 40 to 170 nm, further more preferably from 60 to 135 nm, and even more preferably from 90 to 110 nm.

In the present invention, each oil-in-water emulsified composition is diluted to 20 times to measure the average particle size of the emulsified particle by using Zetasizer Nano Z manufactured by Malvern Panalytical Ltd.

With regard to the oil-in-water emulsified composition of the present invention, from the viewpoint of enhancing the fresh feeling of the skin after application, a viscosity at 25°C is preferably 1 mPa·s or more, more preferably 2 mPa·s or more, further more preferably 5 mPa·s or more, preferably 50 mPa·s or less, more preferably 30 mPa·s or less, and further more preferably 20 mPa·s or less. The viscosity at 25°C is preferably from 1 to 50 mPa·s, more preferably from 2 to 30 mPa·s, and further more preferably from 5 to 20 mPa·s.

In the present invention, the viscosity is measured by using a TVB-10 viscometer with a rotor 1 at 60 rpm at 25°C for 1 minute.

The oil-in-water emulsified composition of the present invention is suitable as an oil-in-water emulsified cosmetic. For example, the composition may be applied as a skin cosmetic such as a skin care cosmetic such as lotion, milky lotion, and beauty essence, and is suitable as lotion having a transparent or translucent appearance.

With regard to the embodiment described above, the present invention further discloses the following composition and the like.

<1> A transparent or translucent oil-in-water emulsified composition comprising following components (A), (B), (C), (D), (E), and (F):

(A) a ceramide;
(B) an anionic surfactant selected from the group consisting of N-acyl-N-alkyltaurine, N-acyl-L-glutamic acid, and salts thereof;
(C) a nonionic surfactant having an HLB of 10 or more;
(D) a dihydric alcohol having 6 to 10 carbon atoms;
(E) a dihydric alcohol having 5 or less carbon atoms; and
(F) water.

<2> The oil-in-water emulsified composition according to the <1>, wherein the ceramide as the component (A) is preferably one or more selected from the group consisting of a natural ceramide, a sphingosine, and the compounds of the formulas (1) and (2), and the composition comprises more preferably one or more selected from the group consisting of the natural ceramide, the sphingosine, and the compound of the formula (1), further more preferably one or more selected from the group consisting of the natural ceramide, phytosphingosine, and N-(hexadecyloxyhydroxypropyl)-N-hydroxyethylhexadecanamide, and even more preferably the N-(hexadecyloxyhydroxypropyl)-N-hydroxyethylhexadecanamide:

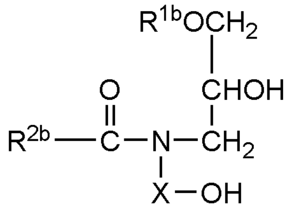

(1)

Wherein $R^{1b}$ represents a hydrocarbon group having 10 to 26 carbon atoms; $R^{2b}$ represents a hydrocarbon group having from 9 to 25 carbon atoms; and X represents - $(CH_2)_n$-, wherein n represents an integer of from 2 to 6.

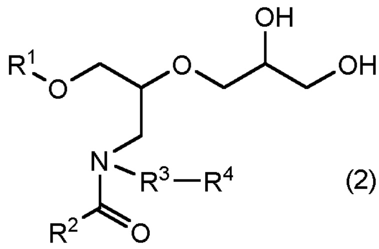

(2)

Wherein $R^1$ and $R^2$ are the same or different and each represent a hydrocarbon group having 1 to 40 carbon atoms which is optionally hydroxylated; $R^3$ represents an alkylene group having 1 to 6 carbon atoms or a single bond; $R^4$ represents a hydrogen atom, an alkoxy group having 1 to 12 carbon atoms, or a 2,3-dihydroxypropyloxy group, provided that $R^4$ is a hydrogen atom when $R^3$ is a single bond.

<3> The oil-in-water emulsified composition according to the <1> or <2>, wherein a content of the component (A) is preferably 0.000 01 mass% or more, more preferably 0.001 mass% or more, further more preferably 0.005 mass% or more, even more preferably 0.03 mass% or more, particularly preferably 0.125 mass% or more, preferably 0.5 mass% or less, more preferably 0.4 mass% or less, further more preferably 0.35 mass% or less, even more preferably 0.27 mass% or less, and particularly preferably 0.23 mass% or less, with respect to the total composition.

<4> The oil-in-water emulsified composition according to any one of the <1> to <3>, wherein the component (B) comprises preferably N-acyl-N-methyltaurine and a salt thereof, more preferably one or more selected from the group consisting of N-lauroyl-N-methyltaurine, N-stearoyl-N-methyltaurine, and salts thereof, further more preferably the N-stearoyl-N-methyltaurine and the salt thereof, and even more preferably sodium N-stearoyl-N-methyltaurine.

<5> The oil-in-water emulsified composition according to any one of the <1> to <4>, wherein a content of the component (B) is preferably 0.001 mass% or more, more preferably 0.015 mass% or more, further more preferably 0.023 mass% or more, even more preferably 0.028 mass% or more, particularly preferably 0.048 mass% or more, preferably 0.5 mass% or less, more preferably 0.35 mass% or less, further more preferably 0.2 mass% or less, even more preferably 0.09 mass% or less, and particularly preferably 0.07 mass% or less, with respect to the total composition.

<6> The oil-in-water emulsified composition according to any one of the <1> to <5>, wherein a mass ratio of the component (A) to the component (B), (A)/(B), is preferably 2 or more, more preferably 2.2 or more, further more preferably 2.7 or more, even more preferably 2.9 or more, preferably 7 or less, more preferably 6 or less, further more preferably 5 or less, and even more preferably 4.5 or less.

<7> The oil-in-water emulsified composition according to any one of the <1> to <6>, wherein the component (C) has an HLB of preferably from 11 to 16, and more preferably from 12 to 15.

<8> The oil-in-water emulsified composition according to any one of the <1> to <7>, wherein the component (C) comprises preferably one or more selected from the group consisting of polyoxyethylene hydrogenated castor oil and polyoxyethylene sorbitan fatty acid ester, and more preferably the polyoxyethylene hydrogenated castor oil.

<9> The oil-in-water emulsified composition according to any one of the <1> to <8>, wherein a content of the component(C) is preferably 0.1 mass% or more, more preferably 0.13 mass% or more, further more preferably 0.2 mass% or more, even more preferably 0.26 mass% or more, preferably 4 mass% or less, more preferably 2 mass% or less, further more preferably 0.7 mass% or less, and even more preferably 0.35 mass% or less, with respect to the total composition.

<10> The oil-in-water emulsified composition according to any one of the <1> to <9>, wherein the component (D) preferably comprises dipropylene glycol.

<11> The oil-in-water emulsified composition according to any one of the <1> to <10>, wherein a content of the component(D) is preferably 0.1 mass% or more, more preferably 0.13 mass% or more, further more preferably 0.15 mass% or more, even more preferably 0.3 mass% or more, preferably 10 mass% or less, more preferably 6 mass% or less, further more preferably 3 mass% or less, and even more preferably 1 mass% or less, with respect to the total composition.

<12> The oil-in-water emulsified composition according to any one of the <1> to <11>, wherein a mass ratio of the component (B) to the component (D), (B)/(D), is preferably 0.005 or more, more preferably 0.01 or more, further more preferably 0.02 or more, even more preferably 0.06 or more, preferably 7 or less, more preferably 1 or less, further more preferably 0.5 or less, and even more preferably 0.2 or less.

<13> The oil-in-water emulsified composition according to any one of the <1> to <12>, wherein the component (E) comprises preferably one or more selected from the group consisting of propylene glycol and 1,3-butylene glycol, and more preferably the 1,3-butylene glycol.

<14> The oil-in-water emulsified composition according to any one of the <1> to <13>, wherein a content of the component(E) is preferably 0.1 mass% or more, more preferably 0.5 mass% or more, further more preferably 1.7

mass% or more, even more preferably 2 mass% or more, preferably 10 mass% or less, more preferably 7.5 mass% or less, further more preferably 2.7 mass% or less, and even more preferably 4 mass% or less, with respect to the total composition.

<15> The oil-in-water emulsified composition according to any one of the <1> to <14>, wherein a total content of the components (D) and (E), (D) + (E), is preferably 0.1 mass% or more, more preferably 1 mass% or more, further more preferably 2.3 mass% or more, even more preferably 2.8 mass% or more, particularly preferably 3 mass% or more, preferably 15 mass% or less, more preferably 12 mass% or less, further more preferably 9 mass% or less, even more preferably 5.5 mass% or less, and particularly preferably 5 mass% or less, with respect to the total composition.

<16> The oil-in-water emulsified composition according to any one of the <1> to <15>, wherein a mass ratio of the component (D) to the component (E), (D)/(E), is preferably 0.01 or more, more preferably 0.02 or more, further more preferably 0.04 or more, even more preferably 0.06 or more, particularly preferably 0.08 or more, more preferably 0.2 or more, preferably 10 or less, more preferably 5 or less, further more preferably 2 or less, even more preferably 1.4 or less, particularly preferably 1 or less, and more preferably 0.56 or less.

<17> The oil-in-water emulsified composition according to any one of the <1> to <16>, wherein a content of the water as the component (F) is preferably 50 mass% or more, more preferably 60 mass%, further more preferably 70 mass% or more, preferably 99 mass% or less, more preferably 95 mass% or less, and further more preferably 90 mass% or less, with respect to the total composition.

<18> The oil-in-water emulsified composition according to any one of the <1> to <17>, further comprising preferably (G) a basic amino acid and more preferably arginine.

<19> The oil-in-water emulsified composition according to the <18>, wherein a content of the component (G) is preferably 0.01 mass% or more, more preferably 0.02 mass% or more, further more preferably 0.05 mass% or more, even more preferably 0.1 mass% or more, preferably 1 mass% or less, more preferably 0.8 mass% or less, further more preferably 0.5 mass% or less, and even more preferably 0.2 mass% or less, with respect to the total composition.

<20> The oil-in-water emulsified composition according to any one of the <1> to <19>, wherein a content of oil components other than the component (A) is preferably 15 mass% or less, more preferably 5 mass% or less, further more preferably 1 mass%, even more preferably 0.5 mass% or less, and particularly preferably 0.1 mass% or less.

<21> The oil-in-water emulsified composition according to any one of the <1> to <20>, wherein an average particle size of the emulsified particle is preferably 10 nm or more, more preferably 40 nm or more, further more preferably 60 nm or more, even more preferably 90 nm or more, preferably 200 nm or less, more preferably 170 nm or less, further more preferably 135 nm or less, and even more preferably 110 nm or less.

<22> The oil-in-water emulsified composition according to any one of the <1> to <21>, wherein a viscosity at 25°C is preferably 1 mPa·s or more, more preferably 2 mPa·s or more, further more preferably 5 mPa·s or more, preferably 50 mPa·s or less, more preferably 30 mPa·s or less, and further more preferably 20 mPa·s or less.

<23> The oil-in-water emulsified composition according to any one of the <1> to <22>, wherein the oil-in-water emulsified composition is preferably an oil-in-water emulsified cosmetic and more preferably an oil-in-water emulsified skin cosmetic.

<24> A transparent or translucent oil-in-water emulsified composition comprising following components (A), (B), (C), (D), (E), and (F):

(A) from 0.000 01 to 0.5 mass% of a ceramide;
(B) from 0.001 to 0.5 mass% of an anionic surfactant selected from the group consisting of N-acyl-N-alkyltaurine, N-acyl-L-glutamic acid, and salts thereof;
(C) from 0.1 to 4 mass% of a nonionic surfactant having an HLB of 10 or more;
(D) from 0.1 to 10 mass% of a dihydric alcohol having 6 to 10 carbon atoms;
(E) from 0.1 to 10 mass% of a dihydric alcohol having 5 or less carbon atoms; and
(F) water.

<25> A transparent or translucent oil-in-water emulsified composition comprising following components (A), (B), (C), (D), (E), and (F):

(A) from 0.000 01 to 0.5 mass% of N-(hexadecyloxyhydroxypropyl)-N-hydroxyethylhexadecanamide;
(B) from 0.001 to 0.5 mass% of sodium N-stearoyl-N-methyltaurine;
(C) from 0.1 to 4 mass % of polyoxyethylene hydrogenated castor oil having an HLB of from 12 to 15;
(D) from 0.1 to 10 mass% of dipropylene glycol;
(E) from 0.1 to 10 mass% of 1,3-butylene glycol; and
(F) water.

<26> A transparent or translucent oil-in-water emulsified composition comprising following components (A), (B), (C),

(D), (E), and (F):

(A) from 0.03 to 0.35 mass% of N-(hexadecyloxyhydroxypropyl)-N-hydroxyethylhexadecanamide;
(B) from 0.001 to 0.2 mass% of sodium N-stearoyl-N-methyltaurine;
(C) from 0.13 to 4 mass% of polyoxyethylene hydrogenated castor oil having an HLB of from 12 to 15;
(D) from 0.15 to 10 mass% of dipropylene glycol;
(E) from 0.5 to 10 mass% of 1,3-butylene glycol; and
(F) water.

<27> A transparent or translucent oil-in-water emulsified composition comprising following components (A), (B), (C), (D), (E), (F), and (G):

(A) from 0.000 01 to 0.5 mass% of a ceramide;
(B) from 0.001 to 0.5 mass% of an anionic surfactant selected from the group consisting of N-acyl-N-alkyltaurine, N-acyl-L-glutamic acid, and salts thereof;
(C) from 0.1 to 4 mass% of a nonionic surfactant having an HLB of 10 or more;
(D) from 0.1 to 10 mass% of a dihydric alcohol having 6 to 10 carbon atoms;
(E) from 0.1 to 10 mass% of a dihydric alcohol having 5 or less carbon atoms;
(F) water; and
(G) from 0.01 to 1 mass% of a basic amino acid.

<28> A transparent or translucent oil-in-water emulsified composition comprising following components (A), (B), (C), (D), (E), (F), and (G):

(A) from 0.000 01 to 0.5 mass% of N-(hexadecyloxyhydroxypropyl)-N-hydroxyethylhexadecanamide;
(B) from 0.001 to 0.5 mass% of sodium N-stearoyl-N-methyltaurine;
(C) from 0.1 to 4 mass% of polyoxyethylene hydrogenated castor oil having an HLB of from 12 to 15;
(D) from 0.1 to 10 mass% of dipropylene glycol;
(E) from 0.1 to 10 mass% of 1,3-butylene glycol;
(F) water; and
(G) from 0.01 to 1 mass% of arginine.

<29> A transparent or translucent oil-in-water emulsified composition comprising following components (A), (B), (C), (D), (E), (F), and (G):

(A) from 0.03 to 0.35 mass% of N-(hexadecyloxyhydroxypropyl)-N-hydroxyethylhexadecanamide;
(B) from 0.001 to 0.2 mass% of sodium N-stearoyl-N-methyltaurine;
(C) from 0.13 to 4 mass% of polyoxyethylene hydrogenated castor oil having an HLB of from 12 to 15;
(D) from 0.15 to 10 mass% of dipropylene glycol;
(E) from 0.5 to 10 mass% of 1,3-butylene glycol;
(F) water; and
(G) from 0.05 to 0.5 mass% of arginine.

<30> A transparent or translucent oil-in-water emulsified composition comprising following components (A), (B), (C), (D), (E), and (F):

(A) from 0.005 to 0.35 mass% of a ceramide;
(B) from 0.001 to 0.2 mass% of sodium N-stearoyl-N-methyltaurine;
(C) from 0.13 to 4 mass% of polyoxyethylene hydrogenated castor oil having an HLB of from 12 to 15;
(D) from 0.15 to 6 mass% of dipropylene glycol;
(E) from 0.5 to 10 mass% of 1,3-butylene glycol; and
(F) water,

wherein a total content of the components (D) and (E), (D) + (E), is from 1 to 12 mass%.
<31> A transparent or translucent oil-in-water emulsified composition comprising following components (A), (B), (C), (D), (E), and (F):

(A) from 0.005 to 0.35 mass% of a ceramide;

(B) from 0.001 to 0.2 mass% of sodium N-stearoyl-N-methyltaurine;
(C) from 0.13 to 4 mass% of one or more nonionic surfactants selected from the group consisting of polyoxyethylene hydrogenated castor oil and polyoxyethylene sorbitan fatty acid ester each having an HLB of from 12 to 15;
(D) from 0.15 to 6 mass% of dipropylene glycol;
(E) from 0.5 to 10 mass% of 1,3-butylene glycol; and
(F) water.

<32> A transparent or translucent oil-in-water emulsified composition comprising following components (A), (B), (C), (D), (E), and (F):

(A) from 0.005 to 0.35 mass% of a ceramide;
(B) from 0.001 to 0.2 mass% of sodium N-stearoyl-N-methyltaurine;
(C) from 0.13 to 4 mass% of one or more nonionic surfactants selected from the group consisting of polyoxyethylene hydrogenated castor oil and polyoxyethylene sorbitan fatty acid ester each having an HLB of from 12 to 15;
(D) from 0.15 to 6 mass% of dipropylene glycol;
(E) from 0.5 to 10 mass% of 1,3-butylene glycol; and
(F) water,

wherein a total content of the components (D) and (E), (D) + (E), is from 1 to 12 mass%.

Examples

Examples 1 to 15 and Comparative Examples 1 to 4

Oil-in-water emulsified compositions (lotions) having the compositions shown in Table 1 were produced. Thereafter, the sizes of the emulsified particles immediately after production were measured, and the storage stability (appearance and transmittance) and the fresh feeling and the smoothness of the skin after application for each composition were evaluated. The results are also shown in Table 1.

(Production method)

Components (A) to (E) and (G) were heated and stirred at 85°C to prepare an oily component dissolved phase. The remaining components were stirred to be mixed at 85°C to obtain an aqueous phase. The aqueous phase was added to the oily component dissolved phase, and then cooled while being stirred to obtain each oil-in-water emulsified composition.

(Evaluation method)

(1) Particle size (immediately after production):

The oil-in-water emulsified compositions were each diluted to 20 times immediately after production to measure the average particle size of the emulsified particle by using Zetasizer Nano Z manufactured by Malvern Panalytical Ltd.

(2) Storage stability (appearance):

80 mL of each oil-in-water emulsified composition was placed in a 110 mL transparent glass container (standard size #11, Tokyo Garasu Kikai Co., Ltd.) and then the container was sealed, which was then stored at each storage temperature for each period of time.

· -20°C (storage for 1 day)
· -5°C (storage for 30 days)
· 50°C (storage for 30 days)

Each oil-in-water emulsified composition immediately after production and after being stored at each storage temperature was allowed to stand at 25°C for 24 hours, and then the appearance was visually evaluated in accordance with the following criteria. Evaluation of 2 or more are determined to be acceptable.

3: The composition is a transparent and homogeneous single layer having no precipitates.
2: The composition has no precipitates when allowed to stand, and has a few precipitates which are visually observed when shaken lightly, but there is no problem in practical use.
1: The composition has precipitates which are clearly observed even when allowed to stand.

(3) Storage stability (transmittance):

Each oil-in-water emulsified composition was stored in the same manner as in the (2) and then the transmittance was measured. The storage was performed at -20°C and 50°C.
By using an ultraviolet-visible Spectrophotometer (UV1800) manufactured by SHIMADZU CORPORATION, the transmittance was calculated as the proportion at which light having a wavelength of 550 nm passed through the oil-in-water emulsified composition (undiluted solution) assuming that the transmittance of pure water was 100%.

(4) Fresh feeling and smoothness of skin after application:

Three expert evaluators applied 60 mg of each oil-in-water emulsified composition to the back of their hands and spread it in a circle having a diameter of 5 cm for 20 seconds. Thereafter, the fresh feeling and the smoothness of the skin after 5 minutes were evaluated in accordance with the following criteria. The results were shown as the total score given by the three evaluators.

(4-1) Fresh feeling:

5: Clearly fresh
4: Fresh
3: Slightly fresh
2: Slightly sticky
1: Clearly sticky

(4-2) Smoothness:

5: Clearly smooth
4: Smooth
3: Slightly smooth
2: Not very smooth
1: Clearly not smooth

[Table 1]

| | Component name | | Example | | | | | | | | | | | | | | | Comparative Example | | | |
|---|---|---|---|---|---|---|---|---|---|---|---|---|---|---|---|---|---|---|---|---|---|
| | | | 1 | 2 | 3 | 4 | 5 | 6 | 7 | 8 | 9 | 10 | 11 | 12 | 13 | 14 | 15 | 1 | 2 | 3 | 4 |
| A | N-(hexadecyloxyhydroxypropyl)-N-hydroxyethylhexadecanamide *1 | | 0.2 | 0.05 | 0.3 | 0.2 | 0.2 | 0.2 | 0.2 | 0.2 | 0.2 | 0.2 | 0.2 | 0.2 | | 0.2 | 0.2 | 0.2 | 0.2 | 0.2 | 0.2 |
| A | Ceramide 2 *2 | | | | | | | | | | | | | | 0.01 | | | | | | |
| B | Sodium N-stearoyl-N-methyltaurine *3 | | 0.06 | 0.02 | 0.1 | 0.036 | 0.08 | 0.06 | 0.06 | 0.06 | 0.06 | 0.06 | 0.06 | 0.06 | 0.004 | 0.06 | | | 0.06 | 0.06 | 0.06 |
| B | N-stearoyl-L-glutamic acid *4 | | | | | | | | | | | | | | | | 0.06 | | | | |
| C | Polyoxyethylene hydrogenated castor oil (60E.O.) *5 | | 0.3 | 0.3 | 0.3 | 0.3 | 0.3 | 0.15 | 3 | 0.3 | 0.3 | 0.3 | 0.3 | 0.3 | 0.3 | | 0.4 | 0.3 | | 0.3 | 0.3 |
| C | Polyoxyethylene sorbitan monostearate (20E.O.) *6 | | | | | | | | | | | | | | | 0.4 | | | | | |
| D | Dipropylene glycol | | 0.8 | 0.8 | 0.8 | 0.8 | 0.8 | 0.8 | 0.8 | 0.2 | 5 | 0.8 | 0.8 | 0.8 | 0.2 | 0.8 | 0.8 | 0.8 | 0.8 | 0.8 | - |
| E | 1,3-butylene glycol | | 2.5 | 2.5 | 2.5 | 2.5 | 2.5 | 2.5 | 2.5 | 2.5 | 2.5 | 1 | 10 | 2.5 | 1 | 2.5 | 2.5 | 2.5 | 2.5 | - | 3.3 |
| G | L-arginine | | 0.12 | 0.12 | 0.12 | 0.12 | 0.12 | 0.12 | 0.12 | 0.12 | 0.12 | 0.12 | 0.12 | | 0.12 | 0.12 | 0.12 | 0.12 | 0.12 | 0.12 | 0.12 |
| | Methylparaben | | 0.2 | 0.2 | 0.2 | 0.2 | 0.2 | 0.2 | 0.2 | 0.2 | 0.2 | 0.2 | 0.2 | 0.2 | 0.2 | 0.2 | 0.2 | 0.2 | 0.2 | 0.2 | 0.2 |
| | Glycerin | | 4 | 4 | 4 | 4 | 4 | 4 | 4 | 4 | 4 | 4 | 4 | 4 | 4 | 4 | 4 | 4 | 4 | 6.5 | 4 |
| | Polyoxyethylene methyl glucoside | | 0.5 | 0.5 | 0.5 | 0.5 | 0.5 | 0.5 | 0.5 | 0.5 | 0.5 | 0.5 | 0.5 | 0.5 | 0.5 | 0.5 | 0.5 | 0.5 | 0.5 | 0.5 | 0.5 |
| | Polyethylene glycol 1540 | | 0.5 | 0.5 | 0.5 | 0.5 | 0.5 | 0.5 | 0.5 | 0.5 | 0.5 | 0.5 | 0.5 | 0.5 | 0.5 | 0.5 | 0.5 | 0.5 | 0.5 | 0.5 | 0.5 |
| | Trimethylglycine | | 3 | 3 | 3 | 3 | 3 | 3 | 3 | 3 | 3 | 3 | 3 | 3 | 3 | 3 | 3 | 3 | 3 | 3 | 3 |
| | Succinic acid | | 0.1 | 0.1 | 0.1 | 0.1 | 0.1 | 0.1 | 0.1 | 0.1 | 0.1 | 0.1 | 0.1 | 0.1 | 0.1 | 0.1 | 0.1 | 0.1 | 0.1 | 0.1 | 0.1 |
| F | Purified water | | Balance | Balance | Balance | Balance | Balance | Balance | Balance | Balance | Balance | Balance | Balance | Balance | Balance | Balance | Balance | Balance | Balance | Balance | Balance |
| | Total | | 100 | 100 | 100 | 100 | 100 | 100 | 100 | 100 | 100 | 100 | 100 | 100 | 100 | 100 | 100 | 100 | 100 | 100 | 100 |
| | Total amount of A | | 0.2 | 0.05 | 0.3 | 0.2 | 0.2 | 0.2 | 0.2 | 0.2 | 0.2 | 0.2 | 0.2 | 0.2 | 0.01 | 0.2 | 0.2 | 0.2 | 0.2 | 0.2 | 0.2 |
| | Total amount of B | | 0.06 | 0.02 | 0.1 | 0.036 | 0.08 | 0.06 | 0.06 | 0.06 | 0.06 | 0.06 | 0.06 | 0.06 | 0.004 | 0.06 | 0.06 | 0 | 0.06 | 0.06 | 0.06 |
| | Total amount of C | | 0.3 | 0.3 | 0.3 | 0.3 | 0.3 | 0.15 | 3 | 0.3 | 0.3 | 0.3 | 0.3 | 0.3 | 0.3 | 0.4 | 0.4 | 0.3 | 0 | 0.3 | 0.3 |
| | Total amount of D | | 0.8 | 0.8 | 0.8 | 0.8 | 0.8 | 0.8 | 0.8 | 0.2 | 5 | 0.8 | 0.8 | 0.8 | 0.2 | 0.8 | 0.8 | 0.8 | 0.8 | 0.8 | 0 |
| | Total amount of E | | 2.5 | 2.5 | 2.5 | 2.5 | 2.5 | 2.5 | 2.5 | 2.5 | 2.5 | 1 | 10 | 2.5 | 1 | 2.5 | 2.5 | 2.5 | 2.5 | 2.5 | 3.3 |
| | (D)+(E) | | 3.3 | 3.3 | 3.3 | 3.3 | 3.3 | 3.3 | 3.3 | 2.7 | 7.5 | 1.8 | 10.8 | 3.3 | 1.2 | 3.3 | 3.3 | 3.3 | 3.3 | 3.3 | 3.3 |
| | (A)/(B) | | 3.3 | 2.5 | 3 | 5.6 | 2.5 | 3.3 | 3.3 | 3.3 | 3.3 | 3.3 | 3.3 | 3.3 | 2.5 | 3.3 | 3.3 | - | 3.3 | 3.3 | 3.3 |
| | (B)/(D) | | 0.075 | 0.025 | 0.125 | 0.045 | 0.1 | 0.075 | 0.075 | 0.3 | 0.012 | 0.075 | 0.075 | 0.075 | 0.02 | 0.075 | 0.075 | 0 | 0.075 | 0.075 | - |
| | (D)/(E) | | 0.32 | 0.32 | 0.32 | 0.32 | 0.32 | 0.32 | 0.32 | 0.08 | 2 | 0.8 | 0.08 | 0.32 | 0.2 | 0.32 | 0.32 | 0.32 | 0.32 | - | - |
| Particle size(nm) | | Immediately after production | 100 | 100 | 100 | 120 | 80 | 100 | 100 | 150 | 100 | 150 | 100 | 150 | 50 | 100 | 120 | 300 | 170 | 200 | 180 |
| Storage stability | Appearance | Immediately after production | 3 | 3 | 3 | 3 | 3 | 3 | 3 | 2 | 3 | 2 | 3 | 2 | 3 | 3 | 3 | 1 | 2 | 2 | 2 |
| Storage stability | Appearance | -20°C (storage for 1 day) | 3 | 3 | 3 | 3 | 3 | 2 | 3 | 3 | 3 | 3 | 3 | 3 | 3 | 3 | 3 | 1 | 1 | 3 | 3 |
| Storage stability | Appearance | -5°C (storage for 30 days) | 3 | 3 | 3 | 3 | 2 | 3 | 3 | 3 | 3 | 3 | 3 | 3 | 3 | 3 | 3 | 2 | 3 | 3 | 3 |
| Storage stability | Appearance | 50°C (storage for 30 days) | 3 | 3 | 2 | 2 | 3 | 2 | 3 | 2 | 2 | 2 | 2 | 2 | 3 | 2 | 2 | 1 | 1 | 1 | 1 |
| Storage stability | Transmittance (%) | Immediately after production | 88 | 95 | 81 | 84 | 92 | 88 | 92 | 77 | 89 | 75 | 91 | 79 | 98 | 88 | 86 | 55 | 60 | 65 | 71 |
| Storage stability | Transmittance (%) | -20°C (storage for 1 day) | 86 | 90 | 74 | 84 | 89 | 41 | 89 | 75 | 88 | 73 | 90 | 78 | 92 | 87 | 86 | 35 | 16 | 77 | 73 |
| Storage stability | Transmittance (%) | 50°C (storage for 30 days) | 71 | 82 | 51 | 60 | 80 | 44 | 86 | 42 | 49 | 43 | 61 | 57 | 80 | 69 | 62 | 24 | 10 | 50 | 50 |
| | Fresh feeling of skin after application | | 15 | 15 | 14 | 15 | 15 | 15 | 11 | 15 | 11 | 15 | 11 | 15 | 15 | 15 | 14 | 15 | 15 | 15 | 15 |
| | Smoothness of skin after application | | 15 | 11 | 15 | 15 | 15 | 15 | 15 | 15 | 15 | 15 | 15 | 15 | 10 | 15 | 15 | 15 | 15 | 15 | 15 |

*1: N-(hexadecyloxyhydroxypropyl)-N-hydroxyethylhexadecanamide; Sphingo Lipid-E (manufactured by Kao Corporation)
*2: ceramide 2; CERAMIDE TIC-001 (manufactured by TAKASAGO INTERNATIONAL CORPORATION)
*3: sodium N-stearoyl-N-methyltaurine; NIKKOL SMT (manufactured by NIPPON SURFACTANT INDUSTRIES CO., LTD.)
*4: N-stearoyl-L-glutamic acid; AMISOFT HA-P (AJINOMOTO HEALTHY SUPPLY CO., INC.)
*5: polyoxyethylene hydrogenated castor oil (60 E.O.); EMANON CH-60 (manufactured by Kao Corporation); HLB: 14
*6: polyoxyethylene sorbitan monostearate (20 E.O.); RHEODOL TW-S120V (manufactured by Kao Corporation); HLB: 14.9

Prescription examples 1 to 2

The oil-in-water emulsified compositions (lotions) having the compositions shown in Table 2 were produced in the same manner as in Examples 1 to 15. The compositions each has a transparent or translucent appearance, and the transmittance immediately after production is as shown in Table 2. All of these oil-in-water emulsified compositions have excellent storage stability at low and high temperatures, and impart a fresh feeling and smoothness to skin after application while preventing deterioration in appearance due to freezing and melting.

[Table 2]

| | Component name | Prescription example 1 | Prescription example 2 |
|---|---|---|---|
| A | N-(hexadecyloxyhydroxypropyl)-N-hydroxyethylhexadecanamide *1 | 0.1 | 0.1 |
| B | Sodium N-stearoyl-N-methyltaurine *3 | 0.025 | 0.03 |
| C | Polyoxyethylene hydrogenated castor oil (60E.O.) *5 | 0.3 | 0.3 |
| D | Dipropylene glycol | 0.4 | 0.4 |
| E | 1,3-butylene glycol | 3 | 3 |
| G | L-arginine | 0.1 | 0.1 |
| | Methylparaben | 0.2 | 0.2 |
| | Glycerin | 3 | 15 |

(continued)

| Component name | | Prescription example 1 | Prescription example 2 |
|---|---|---|---|
| | Allantoin | 0.2 | 0.2 |
| | Polyoxyethylene methyl glucoside | 0.5 | 3 |
| | Polyethylene glycol 1540 | 0.5 | 1.5 |
| | Trimethylglycine | 3 | 3 |
| | Phenoxyethanol | 0.1 | 0.1 |
| | Edetate | 0.05 | 0.05 |
| | Succinic acid | 0.1 | 0.1 |
| | Eucalyptus extract | 1 | 1 |
| | Water-soluble ginger extract | 1 | 1 |
| | Hiba-arborvitae extract | 1 | 1 |
| F | Water | Balance | Balance |
| Total | | 100 | 100 |
| Total amount of A | | 0.1 | 0.1 |
| Total amount of B | | 0.025 | 0.03 |
| Total amount of C | | 0.3 | 0.3 |
| Total amount of D | | 0.4 | 0.4 |
| Total amount of E | | 3 | 3 |
| (D)+(E) | | 3.4 | 3.4 |
| (A)/(B) | | 4 | 3.3 |
| (B)/(D) | | 0.0625 | 0.075 |
| (D)/(E) | | 0.13 | 0.13 |
| Storage stability | Transmittance (%) (immediately after production) | 91 | 92 |

## Claims

1. A transparent or translucent oil-in-water emulsified composition comprising the following components (A), (B), (C), (D), (E), and (F):

   (A) a ceramide;
   (B) an anionic surfactant selected from the group consisting of N-acyl-N-alkyltaurine, N-acyl-L-glutamic acid, and salts thereof;
   (C) a nonionic surfactant having an HLB of 10 or more;
   (D) a dihydric alcohol having 6 to 10 carbon atoms;
   (E) a dihydric alcohol having 5 or less carbon atoms; and
   (F) water.

2. The oil-in-water emulsified composition according to claim 1, wherein a mass ratio of the component (B) to the component (D), (B)/(D), is from 0.005 to 7.

3. The oil-in-water emulsified composition according to claim 1 or 2, wherein a mass ratio of the component (D) to the component (E), (D)/(E), is from 0.01 to 10.

4. The oil-in-water emulsified composition according to any one of claims 1 to 3, wherein a content of the component (D)

is from 0.1 to 10 mass%.

**5.** The oil-in-water emulsified composition according to any one of claims 1 to 4, wherein the component (C) comprises one or more selected from the group consisting of polyoxyethylene hydrogenated castor oil and polyoxyethylene sorbitan fatty acid ester.

**6.** The oil-in-water emulsified composition according to any one of claims 1 to 5, further comprising (G) a basic amino acid.

**7.** The oil-in-water emulsified composition according to any one of claims 1 to 6, wherein a viscosity at 25°C is from 1 to 50 mPa·s.

## INTERNATIONAL SEARCH REPORT

International application No.
**PCT/JP2023/044789**

### A. CLASSIFICATION OF SUBJECT MATTER

***A61K 8/68***(2006.01)i; ***A61K 8/06***(2006.01)i; ***A61K 8/34***(2006.01)i; ***A61K 8/44***(2006.01)i; ***A61K 8/46***(2006.01)i; ***A61K 8/86***(2006.01)i; ***A61Q 19/00***(2006.01)i
FI: A61K8/68; A61K8/06; A61Q19/00; A61K8/46; A61K8/44; A61K8/34; A61K8/86

According to International Patent Classification (IPC) or to both national classification and IPC

### B. FIELDS SEARCHED

Minimum documentation searched (classification system followed by classification symbols)

A61K8/68; A61K8/06; A61K8/34; A61K8/44; A61K8/46; A61K8/86; A61Q19/00

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| X | JP 2013-227294 A (KOSE CORP.) 07 November 2013 (2013-11-07) paragraphs [0012], [0044], [0070], [0090]-[0092], [0108] | 1-7 |
| X | JP 2014-208626 A (KOSE CORP.) 06 November 2014 (2014-11-06) paragraphs [0001], [0040], [0049], [0051], [0065]-[0066] | 1-7 |
| A | JP 2019-119742 A (KAO CORPORATION) 22 July 2019 (2019-07-22) entire text | 1-7 |
| A | JP 2016-222600 A (KAO CORPORATION) 26 December 2016 (2016-12-26) entire text | 1-7 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **01 March 2024** | **12 March 2024** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (July 2022)

**INTERNATIONAL SEARCH REPORT**
**Information on patent family members**

International application No.
**PCT/JP2023/044789**

| Patent document cited in search report | Publication date (day/month/year) | Patent family member(s) | Publication date (day/month/year) |
|---|---|---|---|
| JP 2013-227294 A | 07 November 2013 | (Family: none) | |
| JP 2014-208626 A | 06 November 2014 | (Family: none) | |
| JP 2019-119742 A | 22 July 2019 | US 2021/0059910 A1<br>entire text<br>EP 3733159 A1<br>WO 2019/131987 A1 | |
| JP 2016-222600 A | 26 December 2016 | (Family: none) | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2019119742 A
- JP 2018087149 A
- JP 62228048 A
- JP 63216812 A
- JP 63227513 A
- JP 6429347 A
- JP 6431752 A
- JP 8319263 A